# EUROPEAN PATENT APPLICATION

(11) **EP 4 371 594 A1**
(43) Date of publication of application: **22.05.2024**
(21) Application number: 22841485.0
(22) Date of filing: 15.07.2022
(51) Int. Cl.: A61M 11/06, A61M 31/00

(54) **SPRAY DRUG ADMINISTRATION APPARATUS AND SPRAY DRUG ADMINISTRATION SYSTEM COMPRISING SAID APPARATUS**

(30) Priority: 16.07.2021 CN 202110805974
(71) Applicant: Ding, Yaowu, Taixing, Jiangsu 225400 (CN)
(72) Inventor: Ding, Yaowu, Taixing, Jiangsu 225400 (CN)
(74) Representative: Grünecker Patent- und Rechtsanwälte PartG mbB
(86) International application number: PCT/CN2022/105940
(87) International publication number: WO 2023/284852

(57) **Abstract**

A spray drug administration apparatus includes: a sleeve forming with a lumen that is capable of containing a cylinder of a syringe; and at least one nozzle provided at a front end of the sleeve and being in communication with the lumen of the sleeve. A direction of axis of the nozzle is angled with respect to a direction of axis of the sleeve. A finger plate can be formed at or adjacent to a rear end of the sleeve. Two nozzles can be provided at a front end of the sleeve. The spray drug administration apparatus can realize complete spraying of medicinal liquid, even drug administration, and leakage prevention. And further disclosed is a spray drug administration including this apparatus.

## Description

### TECHNICAL FIELD

The present application relates to a drug administration apparatus, especially to a drug administration apparatus that can be used in engagement with a syringe and delivering drug to human body in spray form.

### BACKGROUND

Syringe is a medical instrument commonly used in medical field, for injecting medicine into human body, so as to carry out operations such as disease treatment, vaccine receiving and so on. At present, there is a spray drug administration apparatus used in engagement with a syringe in the market, wherein after medicinal liquid is sucked into the syringe, the spray drug administration apparatus can be mated with the syringe, then the spray drug administration apparatus is aligned to nasal cavity, mouth or other part of human body that is adapted for administrating drug, the syringe is pushed to atomize the medicinal liquid and deliver the same into human body. In comparison to the conventional way of administrating drug via syringe, the spray drug administration apparatus can evenly deliver the medicinal liquid to desired portion, and can reduce trauma caused to human body.

During application, it is found that the prior art spray drug administration apparatus still presents some problems. For example, when pushing the syringe to deliver medicinal liquid, pressure within the syringe will rapidly increase, causing loosening of connection between the spray drug administration apparatus and the syringe, thus resulting in leakage therebetween, and even worse, the spray drug administration apparatus and the syringe will disengage from each other, even jetting away the spray drug administration apparatus, this will produce potential risk.

For another example, in some applications, such as when spraying to a nasal cavity of the user, the spray drug administration apparatus and the syringe do not orient properly, in which the syringe takes the posture in which its front portion is high and its rear portion is low, thus there is air in the front end of the syringe, then it is air that is firstly jet, then follows the medicinal liquid. Therefore, there will be much medicinal liquid residue in the spray drug administration apparatus and the syringe, which resulting in inaccurate amount of medicine applied, and it also wastes the medicinal liquid.

Besides, during drug administration to nasal cavities, it is needed to administrate drug respectively to two nasal cavities. Such operation is complex, and it is also hard to guarantee that the two nasal cavities are applied with equal amount of medicine, and sometimes there is even a great difference between the amount of the medicine applied to each of the two nasal cavities.

So, there is need to further improve the structure of the spray drug administration apparatus, to overcome the above mentioned technical problem existing in the spray drug administration apparatus in the prior art.

### SUMMARY

The present invention is made for overcoming the existing problems in the prior art mentioned above. The purpose of the present application is to provide a spray drug administration apparatus with improved structure, which can avoid the problem of disengagement or leakage between the spray drug administration apparatus and the syringe when they sleeve to each other. Besides, the present application also realize complete spraying of medicinal liquid in the apparatus and the syringe by improvement made to the structure of the spray drug administration apparatus. Further, the spray drug administration apparatus with improved structure can also realize even administration of medicine to two nasal cavities.

The spray drug administration apparatus of the present invention includes:
a sleeve forming with a lumen that is capable of containing a cylinder of a syringe; and
at least one nozzle provided at a front end of the sleeve and being in communication with the lumen of the sleeve;
wherein a direction of axis of the nozzle is angled to a direction of axis of the sleeve.

In the above mentioned structure, since the direction of axis of the nozzle is identically angled to the direction of axis of the sleeve, it is also angled to a direction of axis of the syringe inserted in the sleeve. Thus, when delivering medicinal liquid to the nasal cavities by using the spray drug administration apparatus, the syringe will take a posture in which its front portion is low and rear portion is high. At this time, the medicinal liquid will be accumulated in the front end of the syringe, and there is gas behind the medicinal liquid. When pushing the medicinal liquid, the gas can be compressed, thus the compression force can provide additional power pushing the medicinal liquid. And, at the end of pushing the medicinal liquid, the compressed gas will function to sweep the medicinal liquid, then completely pushing out the residue medicinal liquid.

In another structure of the present invention, the spray drug administration apparatus includes:
a sleeve forming with a lumen that is capable of containing a cylinder of a syringe;
at least one nozzle provided at a front end of the sleeve and being in communication with the lumen of the sleeve; and
a finger plate formed at or adjacent to a rear end of the sleeve.

The provision of the finger plate can facilitate in keeping the spray drug administration apparatus and the syringe together when pushing out the medicinal liquid, reducing medicinal liquid leakage therebetween, and also preventing the spray drug administration apparatus from being jet.

In a further structure of the present invention, the spray drug administration apparatus includes:
a sleeve forming with a lumen that is capable of containing a cylinder of a syringe; and
two nozzles provided at a front end of the sleeve and positioned respectively at left side and right side of the front end, and the two nozzles being in communication with the lumen of the sleeve.

The provision of two nozzles allows drug administration to both nasal cavities at the same time, thus guaranteeing equal amounts of medicinal liquid delivered to two nasal cavities.

Preferably, the nozzle includes a curved tube, one end of the curved tube is connected at a front end of the spray drug administration apparatus, a nozzle core is inserted in the other end of the curved tube, and a nozzle head is sleeved on the other end of the curved tube. More preferably, the curved tube is a bellows, so that it allows adjustment of curved angle of the curved tube.

Preferably, there can be one nozzle, wherein the nozzle includes a nozzle head and a nozzle core housed in the nozzle head, wherein the nozzle head is integrally formed on the sleeve.

Preferably, the nozzle includes a curved tube and a nozzle core, one end of the curved tube is connected to a front end of the spray drug administration apparatus, a post is formed in the other end of the curved tube, and the nozzle core is sleeved onto the post from the other end of the curved tube. Wherein, the curved tube is preferably rotatable with respect to the sleeve, thus allowing adjustment of an angle of the nozzle to the sleeve. Further preferably, the curved tube can be a bellows, which facilitate in rotation of the curved portion with respect to the sleeve.

Of course, the case that the angle of the nozzle to the sleeve is fixed is also in the scope of the present invention, for example the angle of the nozzle to the sleeve can be fixed by a mating structure of cutout-tab.

Preferably, an observation portion is formed on the sleeve, through which the syringe is visible. Thus, the observation portion can facilitate the user in controlling the amount of medicinal liquid pushed.

Preferably, the observation portion is a hollowed portion or transparent portion formed on the sleeve.

Preferably, the nozzle includes a nozzle head, on which a breathing passageway is formed. The breathing passageway allows the user to breath in air when applying the medicinal liquid, thus facilitating in applying the medicinal liquid more deeply. And, the breathing passageway also allows the user to breath smoothly.

Specifically, the breathing passageway is formed of at least one of the following structure: breathing opening(s) and breathing slot(s).

And further disclosed is a spray drug administration system including the spray drug administration apparatus mentioned above and a syringe, the syringe can be inserted into the lumen of the sleeve of the spray drug administration apparatus.

Preferably, it also includes a stopping clip detachably mounted on a plunger of the syringe.

### BRIEF DESCRIPTION OF THE DRAWINGS

The drawings show preferable and non-limiting structure(s) of the present invention, and the features and advantageous of the present invention can be clearer with reference to the drawings. Wherein:
Fig. 1 shows a perspective view of a spray drug administration apparatus of a first embodiment of the present invention and a syringe used together.
Fig. 2 shows a top view of the spray drug administration apparatus and the syringe shown in Fig. 1.
Fig. 3 schematically shows the process of inserting the syringe into the spray drug administration apparatus.
Fig. 4 is a cutaway view obtained along the line A-A of Fig. 2.
Fig. 5 is a cutaway view obtained along the line B-B of Fig. 2.
Fig. 6a is an explosive perspective view of the spray drug administration apparatus in Fig. 1.
Fig. 6b is a perspective view showing the sleeve with a varied structure.
Fig. 6c shows a perspective view of the curved tube with a varied structure mating with the sleeve of Fig. 6b.
Fig. 7 shows a perspective view of a spray drug administration apparatus of a second embodiment of the present invention and a syringe used together.
Fig. 8 is a cutaway view of the spray drug administration apparatus and the syringe shown in Fig. 7.
Fig. 9 is a perspective view showing the spray drug administration apparatus and the syringe with varied structures of the second embodiment.
Fig. 10 schematically shows the action of the spray drug administration apparatus and the syringe when a stopping clip is mounted.
Fig. 11 shows a perspective view of the stopping clip.
Fig. 12 shows a perspective view of a spray drug administration apparatus of a third embodiment of the present invention and a syringe used together.
Fig. 13 shows an explosive side view of the spray drug administration apparatus in Fig. 12.
Fig. 14 shows a perspective view of a spray drug administration apparatus of a fourth embodiment of the present invention and a syringe used together.
Fig. 15 shows a partial explosive perspective view of the spray drug administration apparatus and the syringe of Fig. 14.
Fig. 16 shows a cutaway view of the spray drug administration apparatus in Fig. 14.
Fig. 17 shows a perspective view of a spray drug administration apparatus of a fifth embodiment of the present invention.
Fig. 18 shows a perspective view of a spray drug administration apparatus of a sixth embodiment of the present invention.
Fig. 19 shows an explosive perspective view of the spray drug administration apparatus in Fig. 18.
Fig. 20 shows a cutaway view of the nozzle in Fig. 18.
Fig. 21 shows a perspective view of a spray drug administration apparatus of a seventh embodiment of the present invention.
Fig. 22a shows a cutaway view of the nozzle head in Fig. 21.
Fig. 22b shows a perspective view of the nozzle head in Fig. 21.
Fig. 23 shows a perspective view of the spray drug administration apparatus shown in Fig. 21 with a varied structure.
Fig. 24a shows a cutaway view of the nozzle head in Fig. 23.
Fig. 24b shows a perspective view of the nozzle head in Fig. 23.
Fig. 25 shows a perspective view of the spray drug administration apparatus shown in Fig. 21 with another varied structure.
Fig. 26a shows a cutaway view of the nozzle head in Fig. 25.
Fig. 26b shows a perspective view of the nozzle head in Fig. 25.

### DETAILED DESCRIPTION

In order to facilitate in understanding the present invention, detailed description on the embodiments of the present invention will be illustrated hereinafter with reference to the drawings. It shall be understood that the drawings only show the preferred embodiments of the present invention, and they shall not be understood as limitations on the scope of the present invention. One skilled in the art can obtain any obvious modifications, variations, equivalents based on the embodiments shown in the drawings, and in case of not conflicting with each other, the technical features in different embodiments described below can be combined with each other at will, and all of these combinations will fall in the scope of the present invention.

In the following detailed illustration on the present invention, terms for directions and orientations such as "front", "rear" and the like are used by referring to the user during usage of the spray drug administration apparatus and the syringe. Wherein, "front" refers to the side away from the user, "rear" refers to the side close to the user.

### <First Embodiment>

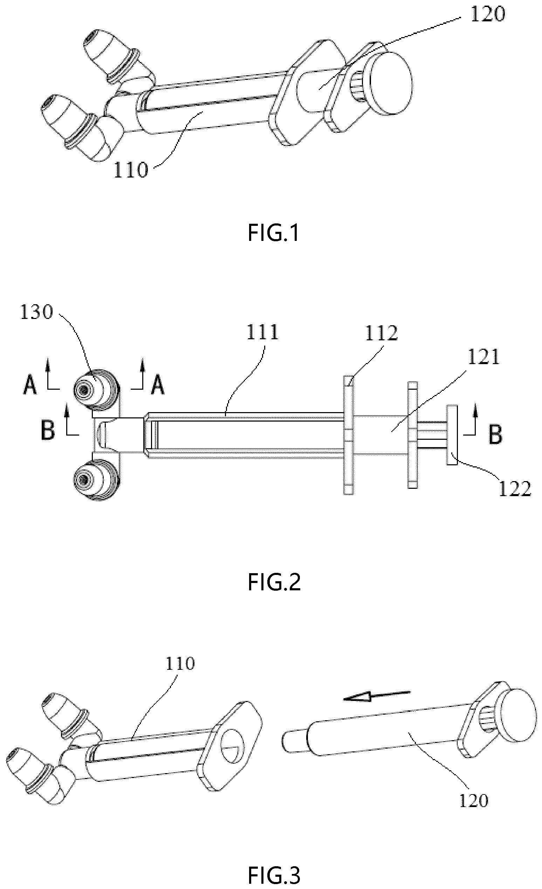

Figs. 1~6 shows a spray drug administration apparatus 110 of a first embodiment of the present invention and a syringe 120 used with the spray drug administration apparatus 110. Fig. 2 shows a top view of an assembly of the spray drug administration apparatus 110 and the syringe 120.

The spray drug administration apparatus 110 includes a sleeve 111, and a cylinder 121 of the syringe 120 can be inserted into a lumen of the sleeve 111, as schematically shown in Fig. 3.

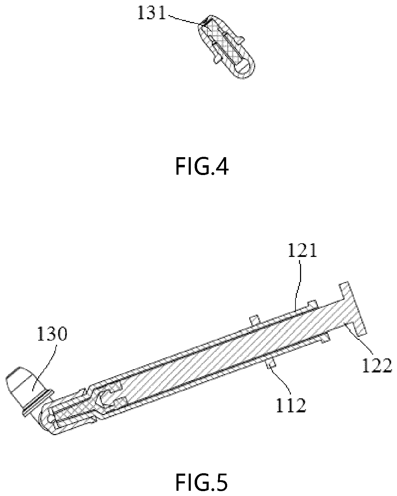

Figs. 4 and 5 respectively show cutaway views obtained along lines A-A and B-B in Fig. 2. As shown in Fig. 4, a nozzle core 131 is provided within the nozzle 130, medicinal liquid from the syringe 120 passes through the nozzle core 131 and is atomized.

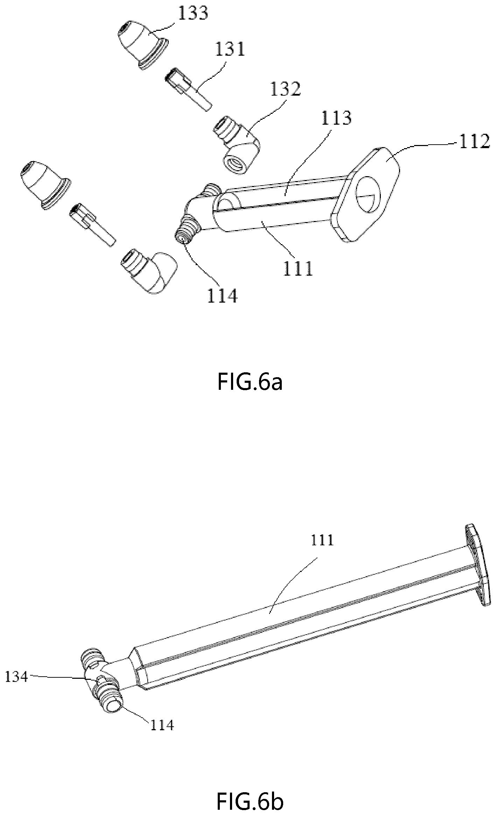

Fig. 6a shows an explosive perspective view of the spray drug administration apparatus 110, which shows the structure of the spray drug administration apparatus 110 in a better way. It can be seen that a finger plate 112 is formed at or near a rear end of the sleeve 111 of the spray drug administration apparatus 110. When the user pushes a plunger 122 of the syringe 120 by a thumb to push the medicinal liquid in the syringe 120, an index finger and a middle finger of the user can pull the finger plate 112. Preferably, a hollowed portion 113 is formed on the sleeve 111, the hollowed portion 113 is provided so that the user can see the scale on the cylinder 121 of the syringe 120 sleeved within the sleeve 111, thus facilitating the user in controlling the amount of the medicinal liquid pushed.

As an alternation to the hollowed portion 113, a portion of the spray drug administration apparatus 110 can be formed of a transparent material, or the spray drug administration apparatus 110 can be formed completely of a transparent material, for example formed of a transparent plastic material and the like.

The finger plate 112 can be formed of any suitable shape, for example polygon, circle and the like, which all fall in the scope of the present invention.

Two nozzles 130 are provided at a front end of the sleeve 111. In detail, the left and right sides of the front end of the sleeve 111 are respectively formed with a side opening 114, in other words, two side openings 114 are formed at the front end. The opening direction of the side openings 114 angles with respect to the direction of axis of the sleeve 111, preferably perpendicular to the direction of axis.

Each side opening 114 is connected with a curved tube 132 of the nozzle 130. For example, a convex ring structure is formed on the side opening, and correspondingly, a slot structure is formed on an inner surface at an end of the curved tube 132 adjacent to the side opening 114. When the curved tube 132 is mounted onto the side opening 114, the convex ring structure and the slot structure form a snap fit, thus realizing connection between the curved tube 132 and the side opening 114. The nozzle core 131 is inserted into the other end of the curved tube 132, a nozzle head 133 covers said the other end of the curved tube 132, thus the nozzle 130 is assembled. As can be more clearly seen from Fig. 5, when assembled, the nozzle 130 is arranged so that its longitudinal axis is angled to the longitudinal axis of the sleeve 111, or in other words, the two longitudinal axes are not in one straight line. And, the snap fit between the convex ring structure and the slot structure allows rotation of the curved tube 132 with respect to the sleeve 111, so that the angle formed by the longitudinal axis of the nozzle 130 with respect to the longitudinal axis of the sleeve 111 can be adjusted.

Connection between the curved tube 132 and the side opening 114 can be realized by other ways, such as by engagement between thread structures and the like. Rotation of the curved tube 132 with respect to the sleeve 111 also can be realized by other additional or alternative structures, for example those described in the embodiments hereinafter.

The curved tube 132 and the side opening 114 can also be non-rotatably fixed to each other.

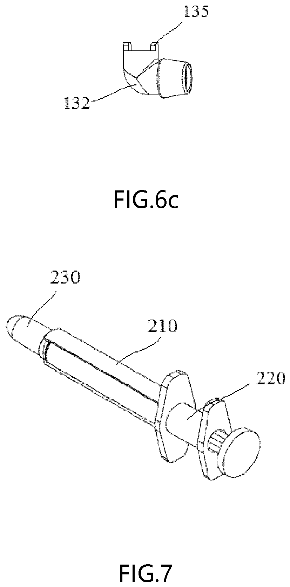

For example, as shown in Figs. 6b and 6c, a cutout 134 is formed at the position of the side opening 114, and a tab 135 is formed on an end of the curved portion 132 connecting to the side opening 114, when the curved tube 132 is connected to the side opening 114, the tab 135 fits into the cutout 134. By means of the interaction between the cutout 134 and the tab 135, the curved tube 132 is prevented from rotation, so that the angle of the curved tube 132 with respect to the sleeve 111 is fixed. The parts where the cutout 134 and the tab 135 are provided can also be interchanged, i.e. the cutout 134 can be formed on the curved tube 132, and the tab 135 can be formed at a corresponding position of the side opening 114.

Then the method of using the spray drug administration apparatus 110 will be described.

First, the user draws medicinal liquid from a medical bottle with the syringe 120. Then the needle of the syringe 120 is removed, and the cylinder 121 of the syringe 120 is inserted into the lumen of the sleeve 111. Then, the user pushes the plunger 122 of the syringe 120, thus pushing the medicinal liquid in the cylinder 121 into the nozzle 130, in which the medicinal liquid is atomized. The medicinal liquid atomized by the nozzle is sprayed out from the nozzle head 133 of the nozzle 130, and can be applied to the patient, such as nasal cavity, mouth and so on.

During pushing the medicinal liquid, the user pushes against the plunger 122, for example by thumb, on one hand, and the finger plate 112 on the sleeve 111 is pulled, for example by index finger and middle finger, on the other hand. In this way, during process of pushing the medicinal liquid, the syringe 120 is kept against the spray drug administration apparatus 110, thus reducing or even eliminating medicinal liquid leakage between the spray drug administration apparatus 110 and the syringe 120, and the spray drug administration apparatus 110 will not be jet by the pressure of the medicinal liquid pushed out.

Further, the spray drug administration apparatus 110 of the first embodiment shown has two nozzles 130, so is particularly adapted to administrate drug to nasal cavities. In other words, when administrating drug, the two nozzles 130 can respectively aligned to the two nostrils of the patient. The two nozzles 130 can be made to be substantially identical, thus when pushing the plunger 122, medicinal liquid can be provided to the two nozzles 130 simultaneously and thus generating atomized medicinal liquid, and the atomized medicinal liquid sprayed can remain substantially identical.

In addition, since the longitudinal axis of the nozzle 130 is angled to the longitudinal axis of the sleeve 111 (also is the longitudinal axis of the syringe 120), when the two nozzles 130 are aligned and inserted into nostrils of the patient, the syringe 120 will take a posture of the front portion being low and the rear portion being high. Thus, when administrating drug, the medicinal liquid will be accumulated at the front end of the syringe 120, and there will be an amount of gas between the medicinal liquid and a front end of the plunger 122. At this time, if the plunger 122 is pushed, the gas between the front end of the plunger 122 and the medicinal liquid will be compressed, the compressed gas will exert an additional push force on the medicinal liquid, thus facilitating in pushing out the medicinal liquid more rapidly. And at the end of the procedure of pushing the drug, compressed air will be pushed out, during the process of which, the pressure of the compressed air can completely sweep out the medicinal liquid residue in the spray drug administration apparatus 110, the syringe 120 and the nozzle 130.

### <Second Embodiment>

Figs. 7∼11 shows the structure and variation of an assembly of the spray drug administration apparatus 210 of the second embodiment of the present invention and the syringe 220. Wherein, features identical or corresponding to those in the first embodiment are designated by identical or similar reference numbers, and the following disclosure will focus on the features different from the first embodiment, and identical features will not be described in detail. Unless otherwise described, the features illustrated in the first embodiment also apply to the second embodiment.

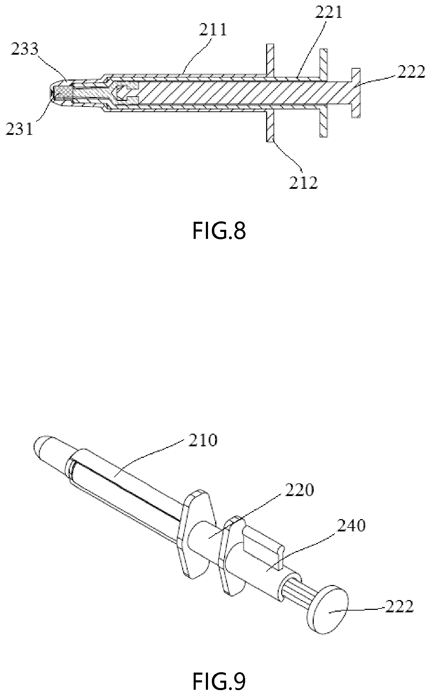

As shown in Figs. 7 and 8, the syringe 220 used with the spray drug administration apparatus 210 includes a single nozzle 230. A longitudinal axis of the nozzle 230 substantially coincides with the longitudinal axis of the spray drug administration apparatus 210, or in other words, they are in one straight line. The nozzle 230 includes a nozzle head 233 and a nozzle core 231 housed in the nozzle head 233. In the preferred structure shown in the drawings, the nozzle head 233 can be integrally formed with the sleeve 211 of the spray drug administration apparatus 210. Of course, the nozzle head 233 and the sleeve 211 can be formed separately, and then assembled together.

Identical to the first embodiment, a finger plate 212 is formed at a rear end of the sleeve 211, when pushing the medicinal liquid in the syringe 220, the user can pull the finger plate 212 at the same time of pushing the plunger 222 of the syringe 220, thus keeping the spray drug administration apparatus 210 and the syringe 220 together.

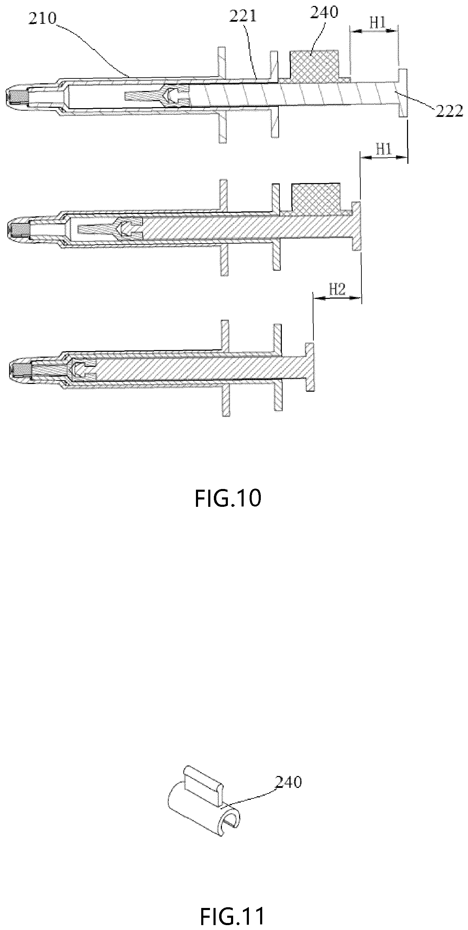

Figs. 9∼11 show schematic view of a variation of the second embodiment of the present invention. Specifically, a stopping clip 240 is mounted on the syringe 220, for assisting in calculating the amount of the medicinal liquid sprayed out. Specifically, the stopping clip 240 is mounted on the plunger 222. Fig. 11 shows an exemplary structure of the stopping clip 240.

As shown in Fig. 10, in an initial position, the length of the plunger 222 exceeds the length of the stopping clip 240 by H1, so it is allowed to push the plunger 222 in a direction towards the front end. After the plunger 222 is pushed forward by a distance of H1, the stopping clip 240 will be seized between the cylinder 221 and the plunger 222, at this time, the stopping clip 240 prevents further pushing forward the plunger 222. Then, drug administration to one nasal cavity is completed. Then, when administrating drug to the other nasal cavity, the stopping clip 240 is removed, so that the plunger 122 can be further pushed in a direction towards the front end, until the medicinal liquid is completely pushed out, for example the plunger 222 is pushed forward by a distant H2, to completely push the medicinal liquid out.

The provision of the stopping clip 240 helps in dividing the medicinal liquid in the syringe 220, so that the amounts of the medicinal liquid delivered to the two nasal cavities are substantially identical.

### <Third Embodiment>

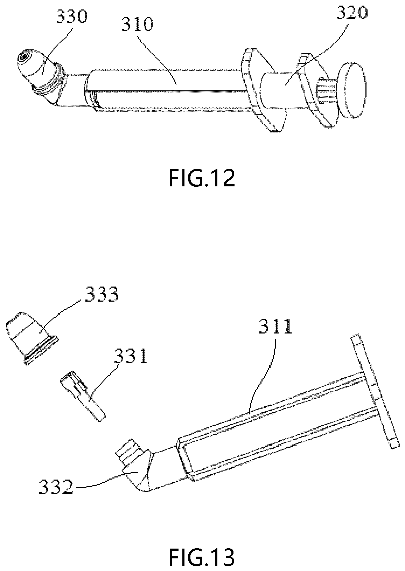

Figs. 12 and 13 show a spray drug administration apparatus 310 of a third embodiment of the present invention and a syringe 320 used therewith. Wherein, features identical or corresponding to those in the first and second embodiments are designated by identical or similar reference numbers, and the following disclosure will focus on the features different from the first and second embodiments, and identical features will not be described in detail. Unless otherwise described, the features illustrated in the first and second embodiments also apply to the third embodiment.

The spray drug administration apparatus 310 of the third embodiment can be used in corporation with the syringe 320, and there is a single nozzle 330 at a front end of the spray drug administration apparatus 310. Different from the second embodiment, the direction of axis of the nozzle 330 of the spray drug administration apparatus 310 in the third embodiment is angled to the direction of axis of the body (i.e. the sleeve) of the spray drug administration apparatus 310, as shown in Fig. 13.

The nozzle 330 also includes a nozzle core 331, a curved tube 332 housing the nozzle core 331 and connected to the body of the spray drug administration apparatus 310, and a nozzle head 333 sleeved on the nozzle core 331 and the curved tube 332.

### <Fourth Embodiment>

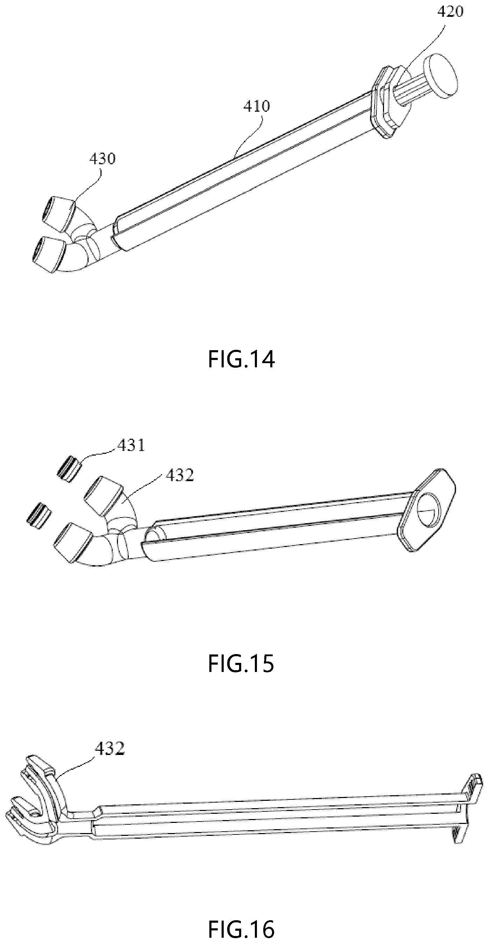

Figs. 14~16 show a spray drug administration apparatus 410 of a fourth embodiment of the present invention and a syringe 420 used therewith. Wherein, features identical or corresponding to those in the first to third embodiments are designated by identical or similar reference numbers, and the following disclosure will focus on the features different from the first to third embodiments, and identical features will not be described in detail. Unless otherwise described, the features illustrated in the first to third embodiments also apply to the fourth embodiment.

In the fourth embodiment, the spray drug administration apparatus 410 includes two nozzles 430, each of which includes a nozzle body 432, and the nozzle body 432 is formed integrally with the spray drug administration apparatus 410, as can be more clearly seen from the cutaway view shown in Fig. 16. Preferably, the nozzle body 432 of the nozzle 430 is generally arc shaped, thus facilitating in manufacturing.

The nozzle 430 also includes a nozzle core 431 inserted into the nozzle body 432.

This structure of integrally formed nozzle 430 facilitates in simplifying the structure and reducing the number of the parts.

### <Fifth Embodiment>

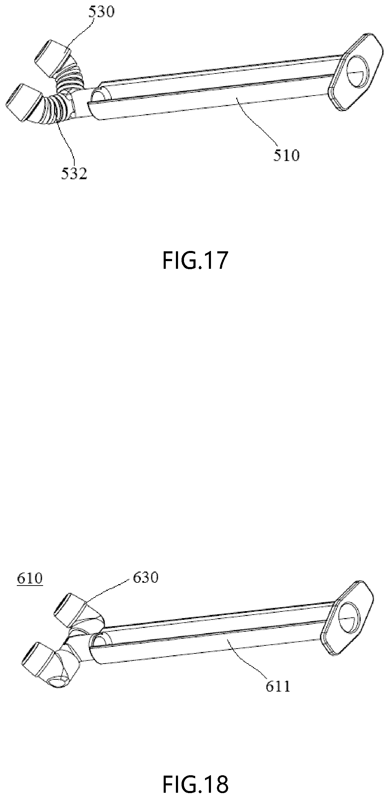

Fig. 17 shows a spray drug administration apparatus 510 of a fifth embodiment of the present invention. Wherein, features identical or corresponding to those in the first to fourth embodiments are designated by identical or similar reference numbers, and the following disclosure will focus on the features different from the first to fourth embodiments, and identical features will not be described in detail. Unless otherwise described, the features illustrated in the first to fourth embodiments also apply to the fifth embodiment.

In the spray drug administration apparatus 510 of the fifth embodiment, the nozzle 530 includes bellows 532 as a nozzle body of the nozzle 530, and the bellows 532 connects to a body of the spray drug administration apparatus 510.

By using bellows 532 as the body of the nozzle, molding can be facilitated, since the bellows 532 can be straight when molding the dame, and can be curved to desired curvature in use as desired. Besides, it is also allowed to adjust curving angle of the bellows 532, so that it can be adapted to multiple applications.

### <Sixth Embodiment>

Figs. 18~20 show a spray drug administration apparatus 610 of a sixth embodiment of the present invention. Wherein, features identical or corresponding to those in the first to fifth embodiments are designated by identical or similar reference numbers, and the following disclosure will focus on the features different from the first to fifth embodiments, and identical features will not be described in detail. Unless otherwise described, the features illustrated in the first to fifth embodiments also apply to the sixth embodiment.

The spray drug administration apparatus 610 includes a sleeve 611 as its body, at a rear end of which a finger plate 612 is formed, and at a front end of which a nozzle 630 is mounted. Similar to the first embodiment, the nozzle 630 can be connected to the sleeve 611 by such means as snap ring-slot (as shown in the drawings), threads connection and so on.

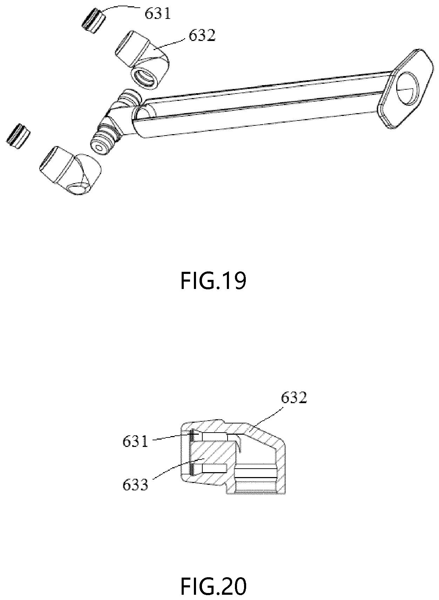

In the sixth embodiment, the nozzle 630 includes a curved tube 632 and a nozzle core 631 inserted in the curved tube 632. Fig. 20 shows a cutaway view of the nozzle 630, wherein it shows that a post 633 is formed in the curved tube 632, the nozzle core 631 inserted in the curved tube 632 can be sleeved on the post 633, thus realizing fixation of the nozzle core 631 on the curved tube 632.

The nozzle 630 with the above mentioned structure can be applied in the spray drug administration apparatus of first to fifth embodiments described above.

### <Seventh Embodiment>

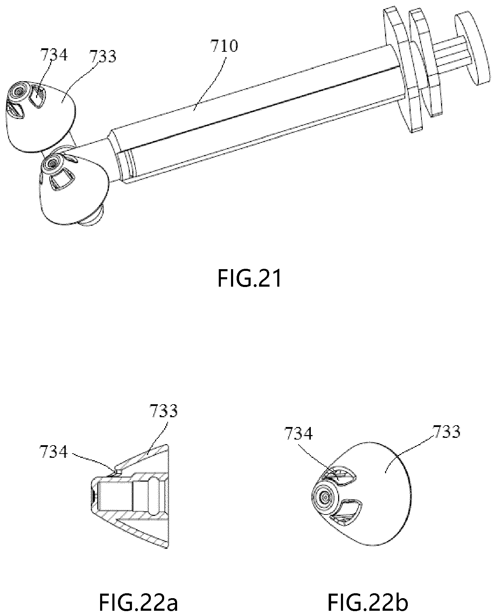

Figs. 21~26b show a spray drug administration apparatus 710 of a seventh embodiment of the present invention. Wherein, features identical or corresponding to those in the first to sixth embodiments are designated by identical or similar reference numbers, and the following disclosure will focus on the features different from the first to sixth embodiments, and identical features will not be described in detail. Unless otherwise described, the features illustrated in the first to sixth embodiments also apply to the seventh embodiment.

As shown in Figs. 21-22b, in the spray drug administration apparatus 710 of the seventh embodiment, a nozzle head 733 of its nozzle is formed with at least one, preferably multiple breathing openings 734. The breathing openings 734 are circumferentially arranged about the nozzle head 733. In one example shown in Figs. 22a and 22b, the breathing opening 734 is generally rectangular.

By means of the provision of the breathing openings 734, when administrating drug to the nasal cavities of the user by the spray drug administration apparatus 710, the breathing openings 734 provides passageway for aspirating air. The aspirated air helps in bringing the atomized medicinal liquid deeply into the nasal cavities, thus facilitating the nasal mucosa in absorbing medicinal liquid. And, the provision of the breathing openings 734 can also keep the user to breath smoothly during drug administration, which is especially useful for kids and so on.

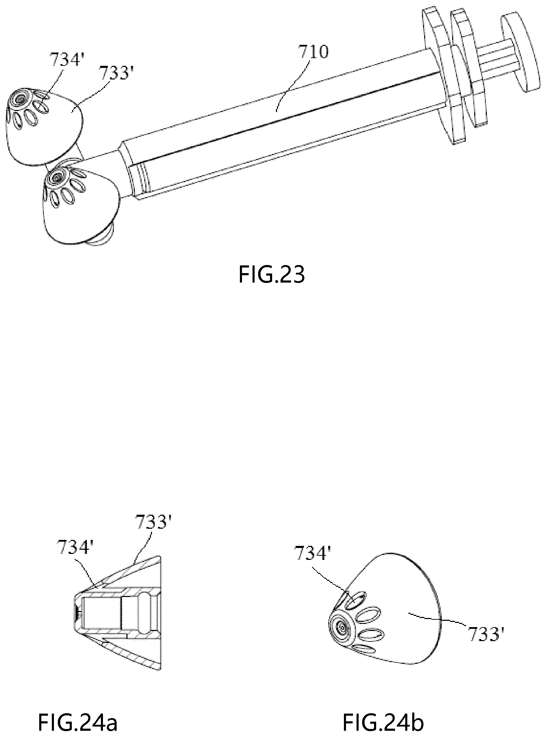

Figs. 23~24b show a variation of the spray drug administration apparatus 710 of the seventh embodiment. Wherein, several circular breathing openings 734' are provided on the nozzle head 733'.

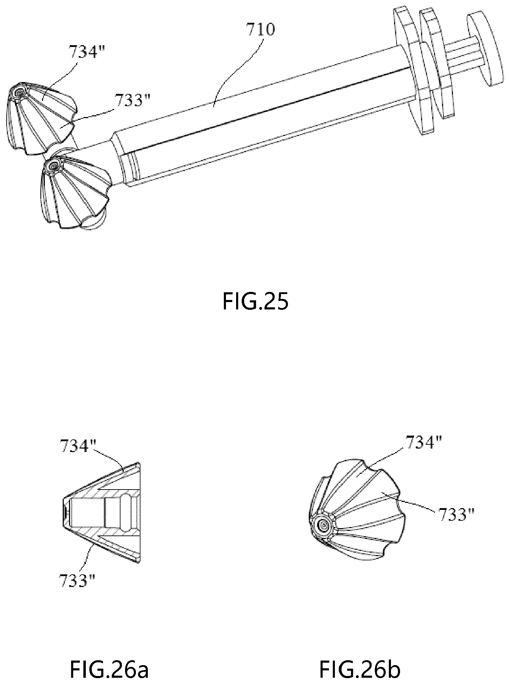

Figs. 25~26b show a further variation of the spray drug administration apparatus 710 of the seventh embodiment. Wherein, a plurality of breathing slots 734" are formed on an outer peripheral surface of the nozzle head 733". These breathing slots 734" extend from a front end of the nozzle head 733" to its rear end. When administrating drug to the nasal cavities of the user, the breathing slots 734" can form passageways for aspiring air.

## Claims

1. A spray drug administration apparatus, **characterized in that** said spray drug administration apparatus includes:
a sleeve forming with a lumen that is capable of containing a cylinder of a syringe; and
at least one nozzle provided at a front end of said sleeve and being in communication with said lumen of said sleeve,
wherein a direction of axis of said nozzle is angled with respect to a direction of axis of said sleeve.

2. A spray drug administration apparatus, **characterized in that** said spray drug administration apparatus includes:
a sleeve forming with a lumen that is capable of containing a cylinder of a syringe;
at least one nozzle provided at a front end of said sleeve and being in communication with said lumen of said sleeve; and
a finger plate formed at or adjacent to a rear end of said sleeve.

3. A spray drug administration apparatus, **characterized in that** said spray drug administration apparatus includes:
a sleeve forming with a lumen that is capable of containing a cylinder of a syringe; and
two nozzles provided at a front end of said sleeve and positioned respectively at left side and right side of said front end, and said two nozzles being in communication with said lumen of said sleeve.

4. The spray drug administration apparatus in accordance with any one of claims 1∼3, **characterized in that** said nozzle includes a curved tube, one end of said curved tube is connected at said front end of said spray drug administration apparatus, a nozzle core is inserted in the other end of said curved tube, and a nozzle head is sleeved on said the other end of said curved tube.

5. The spray drug administration apparatus in accordance with claim 4, **characterized in that** said nozzle is rotatable with respect to said sleeve.

6. The spray drug administration apparatus in accordance with claim 5, **characterized in that** said curved tube is a bellows.

7. The spray drug administration apparatus in accordance with claim 1 or 2, **characterized in that** it includes one said nozzle, wherein said nozzle includes a nozzle head and a nozzle core housed in said nozzle head, wherein said nozzle head is integrally formed on said sleeve.

8. The spray drug administration apparatus in accordance with any one of claims 1∼3, **characterized in that** said nozzle includes a curved tube and a nozzle core, one end of said curved tube is connected to said front end of said spray drug administration apparatus, a post is formed in the other end of said curved tube, and said nozzle core is sleeved onto said post from said the other end of said curved tube.

9. The spray drug administration apparatus in accordance with claim 8, **characterized in that** said curved tube is a bellows.

10. The spray drug administration apparatus in accordance with any one of claims 1∼3, **characterized in that** an observation portion is formed on said sleeve, through which said syringe is visible.

11. The spray drug administration apparatus in accordance with claim 10, **characterized in that** said observation portion is a hollowed portion or a transparent portion formed on said sleeve.

12. The spray drug administration apparatus in accordance with any one of claims 1∼3, **characterized in that** said nozzle includes a nozzle head, on which a breathing passageway is formed.

13. The spray drug administration apparatus in accordance with claim 12, **characterized in that** said breathing passageway is formed of at least one of the following structure: breathing opening(s) and breathing slot(s).

14. A spray drug administration system, **characterized in that** said spray drug administration system includes the spray drug administration apparatus in accordance with any one of claims 1∼13; and a syringe being capable of being inserted into said lumen of said sleeve of said spray drug administration apparatus.

15. The spray drug administration system in accordance with claim 14, **characterized in that** it further includes a stopping clip detachably mounted on a plunger of said syringe.
